# EUROPEAN PATENT SPECIFICATION

(11) **EP 0 552 197 B1**
(45) Date of publication and mention of the grant of the patent: **13.01.1999**
(21) Application number: 91917380.7
(22) Date of filing: 03.10.1991
(51) Int. Cl.: C07D 209/48, C07D 487/04, C08G 59/40, C08J 5/24, C08K 5/3417, C08L 63/00, C08L 63/02

(54) **EPOXY RESINS BASED ON DIAMINOBISIMIDE COMPOUNDS**
EPOXYDHARZE AUF BASIS VON DIAMINOBISIMIDVERBINDUNGEN
RESINES EPOXY A BASE DE COMPOSES DE DIAMINOBISIMIDE

(30) Priority: 03.10.1990 AU 2607/90
(43) Date of publication of application: 28.07.1993
(73) Proprietor: COMMONWEALTH SCIENTIFIC AND INDUSTRIAL RESEARCH ORGANISATION, Campbell, ACT 2601 (AU)
(72) Inventor: HODGKIN, Jonathan, Howard, Burwood, VIC 3125 (AU); JACKSON, Mervyn, Benjamin, Glen Waverley, VIC 3150 (AU); LODER, John, West, South Yarra, VIC 3141 (AU)
(74) Representative: Marshall, Monica Anne
(86) International application number: AU9100454
(87) International publication number: WO9206078

(56) References cited:
- WO-A-93/24488
- US-A- 4 244 857
- CHEMICAL ABSTRACTS, vol. 112, no. 6, 5 February 1990, Columbus, Ohio, US; abstract no. 37255u, R.G. RAJ ET AL. 'Studies on mechanical properties of imide-amine cured epoxy-glass fiber laminates.' page 58 ;column 2 ; & POLYM.-PLAST. TECHNOL. ENG., vol.28, no.5-6, 1989 pages 555 - 566
- JOURNAL OF HETEROCYCLIC CHEMISTRY., vol.15, no.1, January 1978, PROVO US pages 33 - 37 L .C. CASWELL ET AL. 'Cyclic Imides. XI. (1) Bisimide Charge-Transfer Polymers'
- PATENT ABSTRACTS OF JAPAN, C-406, page 163, JP,A, 61225164 (AGENCY OF IND SCIENCE & TECHNOL) 6 October 1986 (06.10.86).
- PATENT ABSTRACTS OF JAPAN, C540, page 150, JP,A, 63150283 (DAINIPPON INK & CHEM INC) 16 December 1986 (16.12.86).
- PATENT ABSTRACTS OF JAPAN, C-453, page 100, JP,A, 62108862 (MITSUI TOATSU CHEM INC) 20 May 1987 (20.05.87).
- PATENT ABSTRACTS OF JAPAN, C-579, page 86, JP,A, 63291956 (UBE IND LTD) 29 November 1988 (29.11.88).
- PATENT ABSTRACTS OF JAPAN, C-105, page 46, JP,A, 57024360 (HITACHI SEISAKUSHO K.K.) 08 February 1982 (08.02.82).
- Polyimides Chap. 1 and 4, D. Wilson, HD Stenzenberger, P Hergenrother Eds, Blackie & Son Glasgow, London 1990;
- Polymer Preprints, 29, No. 1,p. 323

## Description

This invention relates to diaminobisimide compounds suitable for use as curing agents in epoxy resins and to polymer matrices with high glass transition temperatures produced from the cured resins.

The so-called "epoxy resins" are a well known class of thermosetting resins which are prepared by reacting polyepoxides with a curing agent. A large variety of polyepoxides and curing agents are known and have been described in the literature. Epoxy resins are the most widely used resins in the production of advanced composites in which reinforcing fibres, especially carbon fibres, are coated with a formulation comprising a polyepoxide and a curing agent followed by curing to form a composite material. The preferred curing agents for such systems are aromatic diamines. Many of the cheaper aromatic diamines such as
p-phenylene diamine, m-phenylene diamine, 4,4'-diaminodiphenylether, 4,4'-diaminophenylmethane and 4,4'-diaminodiphenylsulphone (DDS) are either oxidatively unstable or cause health and safety problems. The instability problems are generally caused by the conjugation between the two aromatic amino groups and commercial considerations have made it necessary to use less conjugated but high melting materials such as DDS.

A major problem in the advanced materials industry is that the use of epoxy resin formulations as either adhesives or composites involves hand fabrication and requires long and involved procedures to cure. The current toxicity and stability problems of available diamines, such as m-phenylene diamine and diaminodiphenylmethane result in the use of insoluble, expensive and less processable diamines, such as DDS.

In attempts to improve the stability, decrease toxicity and improve the physical properties of these diamines, considerable research has recently gone into the synthesis of many high molecular weight diamines which are less conjugated, such as, for example, Shell Epon HPT 1062, shown below. This has been especially common in the area of epoxy resin hardeners (D A Scola, *Advances in Epoxy Resins*, **4**, 165 (1984)) and in polyimides (T Takeoshi, *Advances in Polymer Science* **94**, 1 (1990)). Unfortunately, the methods of preparing these materials generally involve multistep reactions from costly starting materials or require thermally unstable quaternary carbon compounds or other chemically unsatisfactory atoms in the main chain.

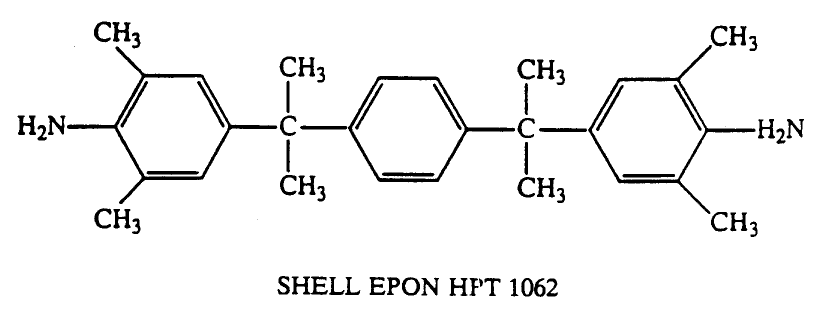

Recently, the synthesis of compounds with bisimide groups between reactive aromatic diamines has been reported, such as in J H Hodgkin, *J Polymer Science:Polymer Chem Ed* **14**, 409 (1976). As the imide group is common in very thermally stable polymers, these diaminobisimides have the potential to be useful diamine monomers. However, apart from those made by D A Scola they are primarily high melting, insoluble materials. Scola's compounds have been tested as hardeners for epoxy resins. They were prepared by reacting aromatic diamines with a fluoroalkylidene aryl dianhydride to give the complex mixture of oligomeric compounds shown in Reaction Scheme 1 below. The complexity of the mixture is acknowledged by these research workers as resulting from multiple reactions of the starting aromatic diamines. As a consequence of this oligomer formation, the fluoroalkylidene aryl dianhydride shown in Reaction Scheme 1 is the only compound which can provide soluble and useful mixtures of hardener materials.

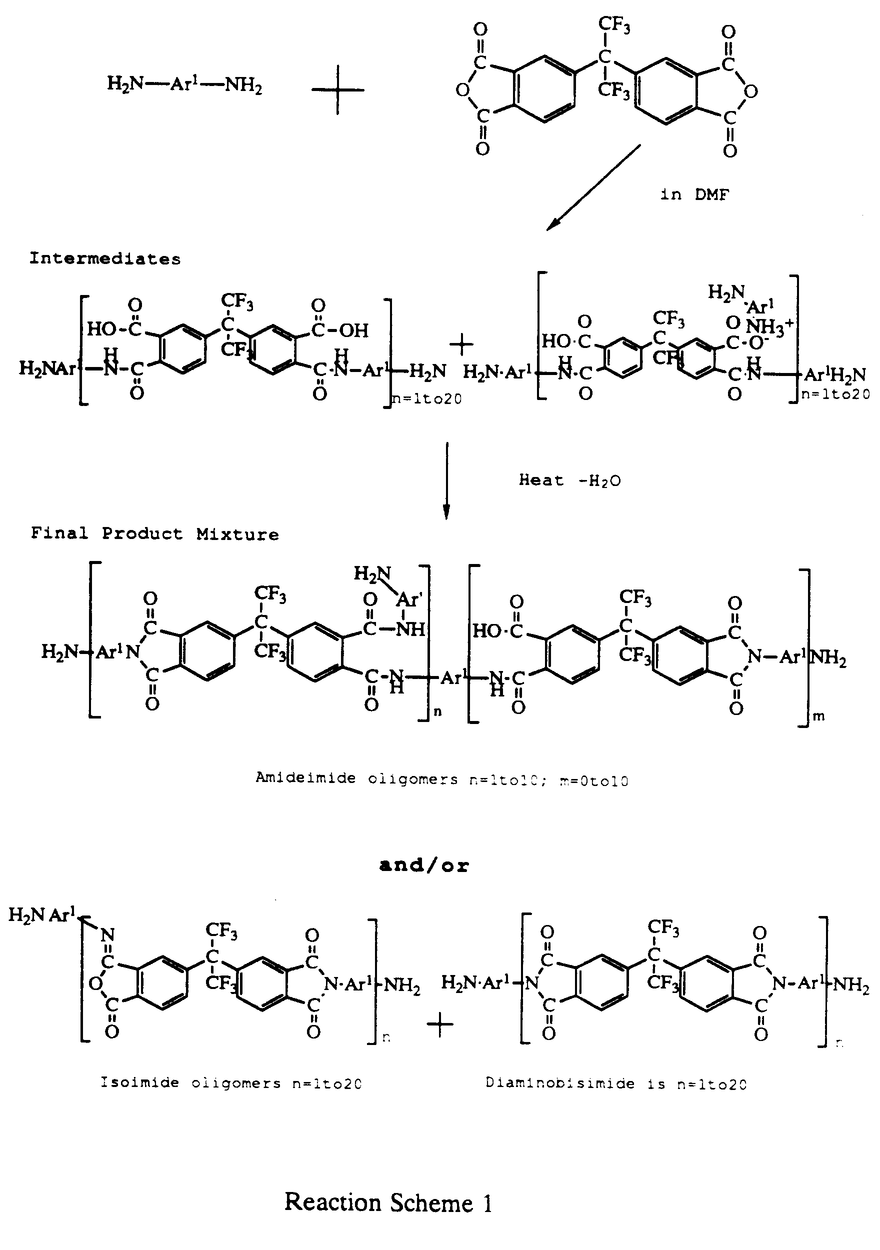

Since purification of such mixtures is extremely difficult, they are used while contaminated with the oligomeric compounds and consequently produce cured resins with properties inferior to those expected if the pure diamine was used. Thus, the synthesis of diaminobisimide compounds of the formula (I) as shown below would be of great commercial utility.

US-A-4244857 discloses the preparation of an aromatic bis(amino-imide) for use as a curing agent for polyepoxides by the reaction of an aryldiamine with an aryl dianhydride or a diester-diacid of such an anhydride. Chemical Abstracts, 112, 1990, No. 37255U relates to a study on the mechanical properties of imide-amine cured epoxy-glass fibre laminates. Neither of these references discloses the preparation of a diaminobisimide compound by reaction with an aryltetracarboxylic acid or an aryldianhydride in the presence of 0.1 to 2 molar proportions of a tertiary amine, or by reaction of the aryldiamine with a higher melting aromatic dianhydride in the molten state such that the reacting diamine melt is always in high molar excess.

Journal of Heterocyclic Chemistry, 15, 1978, pages 33 to 37 describes the condensation of two equivalents of an aromatic cyclic anhydride with an aromatic diamine or two equivalents of an aromatic amine with an aromatic bisanhydride.

We have now found that diaminobisimide compounds of the formula (I) can be prepared in one step and substantially free from oligomers and amide impurities by the use of carefully controlled reaction conditions.

The standard method of synthesizing polyimides used in the plastic industry involves reacting one mole of a pure diamine with one mole of a purified dianhydride in dry and highly purified polar solvents such as dimethylformamide, dimethylacetamide or N-methylpyrrolidone at room temperature and then heating the resulting polyamidoacids to above 180°C to complete the cyclization.

The most successful previous method for synthesizing diaminobisimides is that described by N K Dorogora, *et al*., in which one mole of dianhydride (PMDA or BTDA) in dry dimethylformamide was added slowly to two moles of the diamine in the same solvent at 130°C. The optimum yield obtained was 70% for m-phenylenediamine.

The present invention provides a new method for preparing diaminobisimide compounds of the formula (I) substantially free of oligomers. The products of this method have improved stability and processing properties.

According to one aspect of the invention there is provided a method for the preparation of a diaminobisimide compound of the formula (I) substantially free of oligomers: 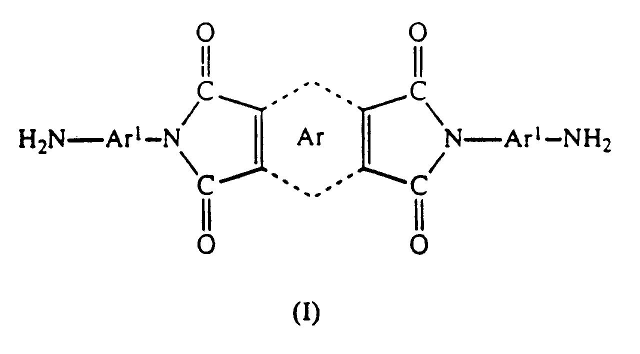 wherein
Ar¹ is an optionally substituted aromatic residue which provides for good conjugation between the nitrogen-containing groups; and
Ar is an optionally substituted aromatic residue,
characterized in that at least two molar proportions of an aromatic diamine of the formula (II)

H₂N-Ar¹-NH₂ (II)

wherein Ar¹ is as defined above
are heated with one molar proportion of an aromatic tetracarboxylic acid of the formula (III), or the corresponding dianhydride,

(HOOC)₂Ar(COOH)₂ (III)

wherein Ar is as defined above,
optionally in the presence of a polar solvent and optionally including 0.1 to 2 molar proportions of a tertiary amine,
with the proviso that when a dianhydride is used either:
(i) 0.1 to 2 molar proportions of a tertiary amine are included, or
(ii) the aromatic diamine is reacted with a higher melting aromatic dianhydride in the molten state such that the reacting diamine melt is always in high molar excess.

As used herein the term "good conjugation" means that during imide formation from the diamine of formula (II), substitution of an electron-withdrawing group on one of the nitrogen atoms suppresses the reactivity of the other nitrogen atom during the reaction.

Suitable Ar groups are aryl, bridged or bonded di- or poly- aryl, and heteroaryl. The Ar groups may be substituted with one or more alkyl, alkoxy, alkylthio, aryl, heteroaryl, aryloxy, carboxy, alkylthio, alkylamino, dialkylamino, amino, nitro, cyano or halo groups.

"Aryl" means an aromatic carbocyclic group, such as phenyl, naphthyl, and the like.

"Bridged or bonded di- or poly- aryl" means a group consisting of two or more aromatic carbocyclic ring systems, such as phenyl, naphthyl or the like joined by a bond, such as in biphenyl, or a bridging group, such as in sulphonyldiphenyl.

"Bridging group" includes for example CF₃-C-CF₃, SO₂, CO and O such as in compounds of the formula (IVa) 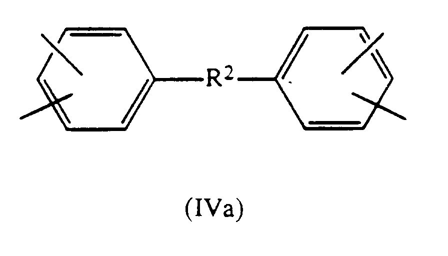 wherein R² is a divalent radical such as CF₃-C-CF₃, SO₂, CH₂ CO and O.

Generally the group Ar¹ may be selected from the groups listed above for Ar. However, because of the constraints imposed by the requirement of "good conjugation" (as defined above) some bridged di- or poly- aryl groups may not be suitable.

Thus for Ar¹, the bridging group (if present) must provide good conjugation between the amino groups of the diamine (II).

For example for groups of the formula (IVb) wherein R¹ is CH₂ or where the diamine is 3,3'-sulphonyldianiline, there is insufficient conjugation and oligomeric diaminoimides are formed. In contrast, benzidine and 4,4'-sulphonyldianilines have sufficient conjugation and give the desired diaminobisimide compound.

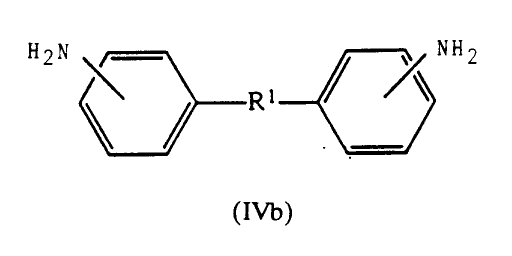

"Heteroaryl" means aromatic monocyclic or polycyclic groups containing at least one hetero atom such as nitrogen, oxygen or sulfur.

Examples of suitable "heteroaryl" groups are:
3- to 8-membered, more preferably 5- or 6-membered, heteromonocyclic groups containing 1 to 4-nitrogen atom(s), for example, pyrrolyl, imidazolyl, pyrazolyl, pyridyl, pyrimidyl, pyrazinyl, pyridazinyl, triazinyl;
condensed heterocyclic groups containing 1 to 5 nitrogen atom(s), for example, indolyl, isoindolyl, indolizinyl, benzimidazolyl, quinolyl, isoquinolyl, indazolyl, benzotriazolyl, etc.;
3 to 8-membered heteromonocyclic groups containing 1 or 2 sulfur atom(s) and 1 to 3 nitrogen atom(s), for example, thiazolyl, isothiazolyl, thiadiazolyl, etc.;
3 to 8-membered heteromonocyclic groups containing 1 or 2 sulfur atom(s), for example thienyl, etc.;
condensed heterocyclic groups containing 1 or 2 sulfur atom(s) and 1 to 3 nitrogen atom(s), for example, benzothiazolyl, benzothiadiazolyl, etc.;
3 to 8-membered heteromonocyclic groups containing an oxygen atom, for example, furyl, etc.;
condensed heterocyclic groups containing 1 to 2 sulfur atom(s), for example, benzothienyl, etc.; and
condensed heterocyclic groups containing 1 or 2 oxygen atom(s), for example, benzofuranyl, etc.

"Alkyl" groups may be straight chain or branched and contain 1-20 carbon atoms. Suitable alkyl groups are methyl, ethyl, propyl, *iso*-propyl, *n*-butyl, *iso*-butyl, *tert*-butyl, *n*-pentyl, *iso*-pentyl, *neo*-pentyl, *n*-octyl, *iso*-octyl, decyl, cetyl, stearyl, and the like.

"Alkoxy" and "alkylthio" mean such groups in which the alkyl moiety is a branched or unbranched saturated hydrocarbon group containing from one to eight carbon atoms, such as methyl, ethyl, propyl, *iso*-propyl, *n*-butyl, *iso*-butyl, *tert*-butyl and the like.

"Alkanoyl" may be formyl, acetyl, propionyl, butyryl, valeryl, isovaleryl, pivaloyl, hexanoyl, and the like.

Preferably the aromatic diamine of the formula (II) is sterically hindered, such as in compounds of the formula (V) 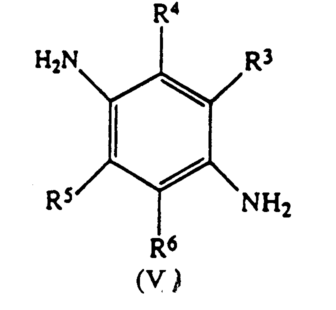 wherein R³, R⁴, R⁵ and R⁶ are the same or different and each may be selected from alkyl, aryl, heteroaryl, nitro and halogen groups.

The present invention also provides a diaminobisimide compound of the formula (I) whenever prepared by the method defined above.

An important aspect of the method of the invention is the use of group conjugation which can be assisted by steric hindrance to achieve monosubstitution in aromatic diamines where the two amino groups originally have similar reactivities. This type of monosubstitution is regularly used in many other areas of chemistry, for example in diisocyanate chemistry for polyurethane synthesis and in the Mannich reaction synthesis of aminomethyl substituted pharmaceuticals (eg. Tramontini, *Synthesis*, (1973) 703), but it is not well known in aromatic diamine synthetic reactions. It has not been possible previously to use these methods for diaminobisimide synthesis as the intermediate product of the reaction is a diaminodiacid of the Formula A as shown below and the free acid groups react with the end amino groups to form oligomeric amides, amideimides, etc., as in Reaction Scheme 1.

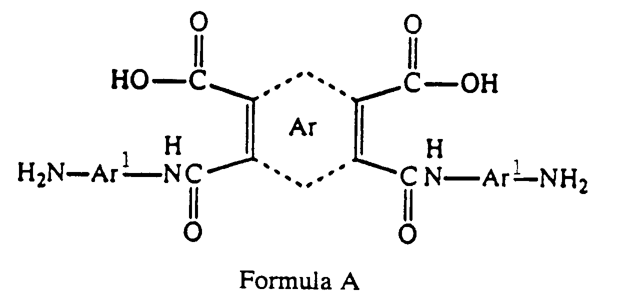

It has been found that for less conjugated amines it is possible to use the endothermic reaction of an aromatic tetracarboxylic acid with the aromatic diamine as shown in Reaction Scheme 2 to inhibit oligomer formation. It has been found that when one mole of the tetracarboxylic acid is mixed with two moles of the diamine, due to ionic repulsion in aqueous solutions or even in relatively non-polar solvents such as acetone only one of the amino groups attaches to a carboxylic acid group (not the two ortho groups).

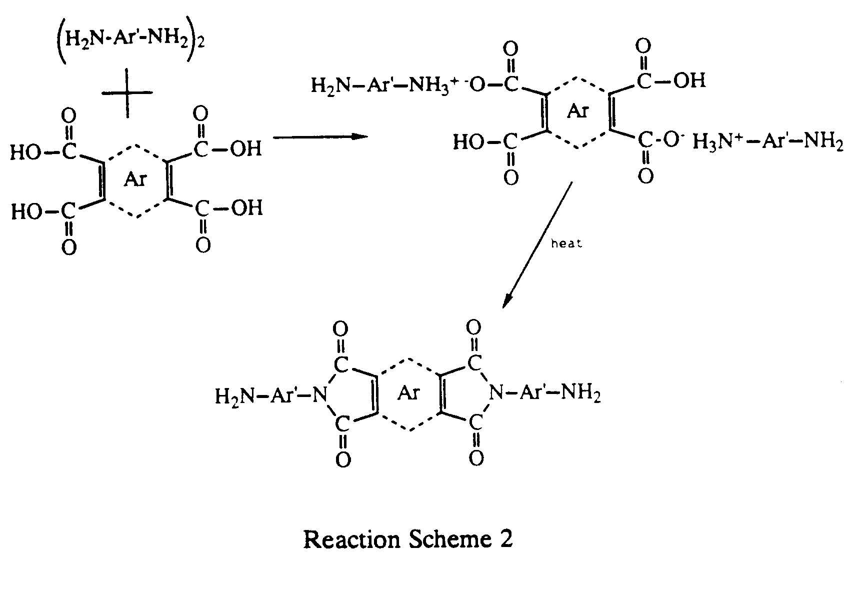

The protonation of one of the amino groups in conjugated diamines depresses the protonation of the second amino group and hence prevents oligomeric salt formation (as seen by NMR and FTIR evidence and pKa measurements). When these salts are heated further (100°-250°C) they cyclize directly to the monomeric diaminobisimides in high yields.

The use of group conjugation in synthesis of one particular series of diaminobisimides was reported by J H Hodgkin, *J Polymer Science:Polymer Chemistry Ed*. Vol 14, **409** (1976) where the tetracarboxylic dianhydride used was 1,4,5,8-naphthalene tetracarboxylic dianhydride. The pure products of the reaction were of the Formula B

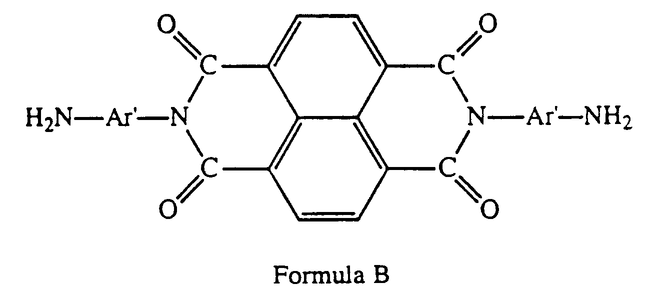

In this reaction, the steric constraints of the adjacent peri carboxyl groups meant that the intermediate diaminodiacids either could not form or were so unstable that the acid groups could not react independently. This is not the case with other dianhydrides, especially the commercially important five membered ring anhydrides such as PMDA, BTDA or 6FDA.

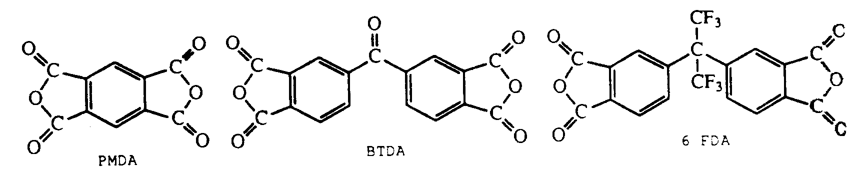

Where a dianhydride is used in the method of the invention it is preferred to add a strong base tertiary amine such as diaminobicyclooctane (DABCO) to the initial primary aromatic diamine solution in sufficient quantity to form a blocking salt with the free acid sites of the diamidodiacid until it is incorporated into the cyclized imide as shown in Reaction Scheme 3. The yield of the above reaction increased to 95% by this method.

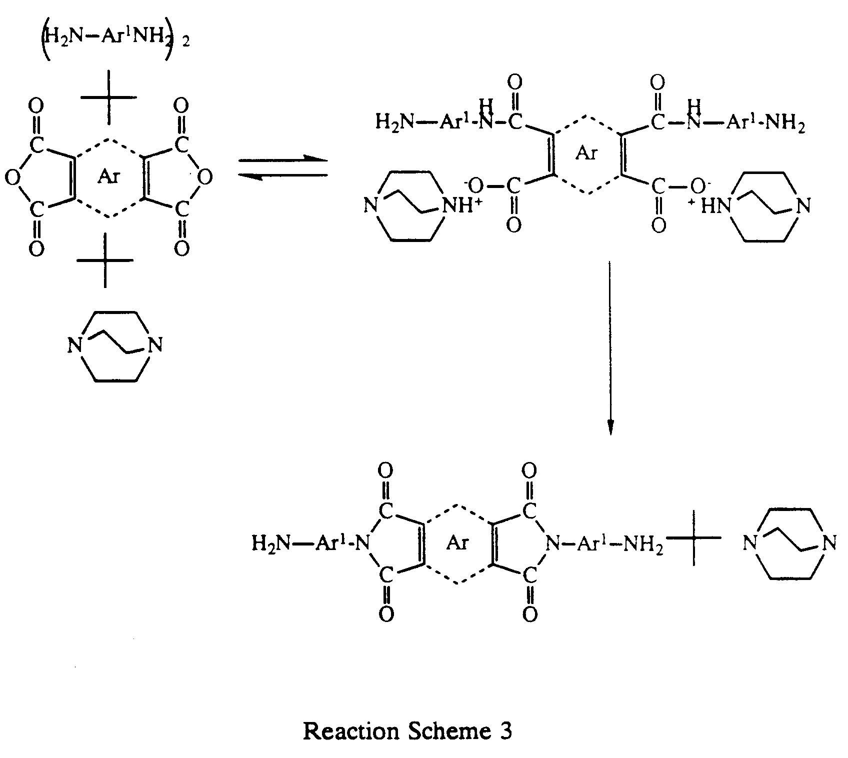

When the addition of the dianhydride is carried out at temperatures above the cyclization temperature of the amidoacids, preferably 120 to 200°C, relatively small amounts of the tertiary amine base are generally required.

As the reaction of an anhydride with a diamine is an exothermic reaction, this method only gives high yields of the pure diaminobisimide with strongly conjugated diamines, that is only when the second amine loses considerable reactivity once the first amine has reacted. This has been found especially when steric hindrance, for example ortho aliphatic groups around the amines, is combined with conjugation to enhance the reactivity changes.

It has been found that when tetracarboxylic acids are used the diamine salts may be formed in a number of different solvents, such as, for example, acetone, wet dimethylformamide and dimethylacetamide. However, the use of water provides considerable advantages of cost reduction and ease of removal from the final product unlike the expensive and troublesome polar solvents which often complex with the final product.

The final cyclization may be carried out by heating, preferably between 80° and 250°C, the dry salt or a water solution or slurry under pressure which contrasts with most previous literature reports suggesting that imides are readily hydrolysed in water.

It has been further shown that in the case of aromatic diamines that are both conjugated and sterically hindered it is possible to form high yields of the diaminobisimide compounds of the formula (I) preferably by heating a molten mixture of the diamine and dianhydride together, provided the diamine melts first and hence is always in much higher concentration in the reaction zone. Again steric hindrance, conjugation and a sufficiently high temperature is generally required to obtain cyclization of the intermediate diaminodiacid without formation of multiamido compounds or oligomers. Similar conditions may be used to form diaminobisimides from molten, sterically hindered, conjugated aromatic diamines and aromatic tetracarboxylic acids. The reaction in this case is via the amine/acid salts as shown above in Reaction Scheme 2.

Sterically hindered amines can yield diaminobisimide compounds which are soluble in relatively non polar solvents, such as, for example, acetone, dichloromethane, chloroform and tetrahydrofuran. This may be contrasted with previously reported diaminobisimide compounds which are high melting insoluble materials except for those mixed oligomeric materials produced from the costly hexafluoroanhydride shown in Formula 1.

The diaminobisimide compounds of the present invention which are generally substantially free of oligomers are useful as thermally stable hardeners for advanced epoxy resin formulations and composites based on such formulations.

Thus, according to another aspect of the present invention there is provided a diaminobisimide compound of the formula (I) as defined above whenever prepared by the method of the invention for use as a curing agent (hardener) in an epoxy resin formulation.

According to a still further aspect of the present invention there is provided a diaminobisimide compound of the formula (I) for use in the manufacture of high temperature resistant thermoplastic and thermoset polymeric materials.

Some of the diaminobisimide compounds of the formula (I) are novel and form a further aspect of the present invention.

Thus, according to a further aspect of the present invention there is provided a diaminobisimide compound of the formula (Ia) 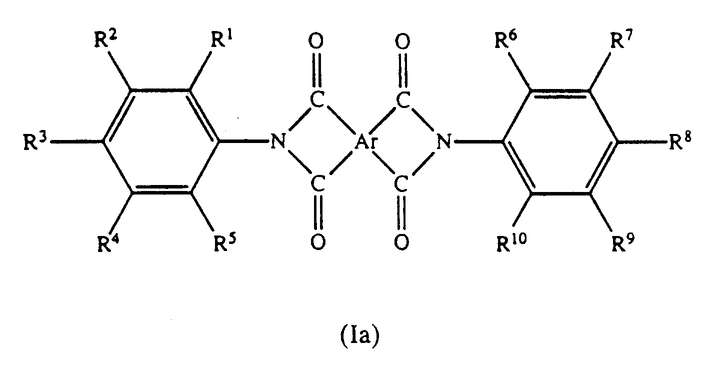 wherein
R¹ to R¹⁰ are the same or different and each may be selected from hydrogen. alkyl, thioalkyl, alkoxy, dialkylamino, alkylamino and amino; and
Ar is as defined above;
with the proviso that at least one of R¹ to R⁵ and R⁶ to R¹⁰ is an alkyl group and at least one of R² to R⁴ and R⁷ to R⁹ is an amino group.

The diaminobisimide compound of the formula (I) is suitable for use as a curing agent (hardener) in an epoxy resin formulation.

The present invention further provides an epoxy resin formulation comprising a mixture of a curing agent and one or more polyepoxides, characterized in that the curing agent is a compound of the formula (I) as defined above.

Any suitable polyepoxides or mixtures thereof may be used in the resin formulations of the invention. The most readily available are N,N,N',N'-tetraglycidyl methylene dianiline (TGDDM) (e.g. Ciba Geigy MY720) and diglycidyl ethers of bisphenol A (DGEBA). For advanced composites, greater than 50 weight % of MY720 is preferred.

Particularly suitable polyepoxides are those prepared from "upper rim" calixarenes as described in our copending Australian Patent Applications Nos. PK 2610/90 and PK 3871/90.

The resin formulations of the invention may also contain various toughening polymers which may be either elastomers or thermoplastics and catalysts.

These resin formulations are easy to process and produce materials with improved properties such as higher Tg, toughness, toughenability and water resistance with less health hazards.

Still further according to the present invention there is provided an impregnated fibre reinforced material (prepregs), characterised in that the fibre reinforcements are coated with the epoxy resin formulation defined above.

In an additional aspect of the present invention there is provided an advanced composite material comprising an assembly of reinforcing fibres in a matrix of cured epoxy resin, characterized in that the cured epoxy resin is formed by heating the epoxy resin formulation as defined above.

Alkylated aromatic diaminobisimides of the formula (Ia) prepared from pyromellitic acid and Ethacure 100 (Formula Ib), referred to herein as CBH-103, and benzophenone tetracarboxylic acid and Ethacure 100 (Formula Ic), referred to herein as CBH-104 have been found to be easily made from these readily available starting materials and give cured epoxy resins and laminates with good properties. ("Ethacure" is a Registered Trade Mark). 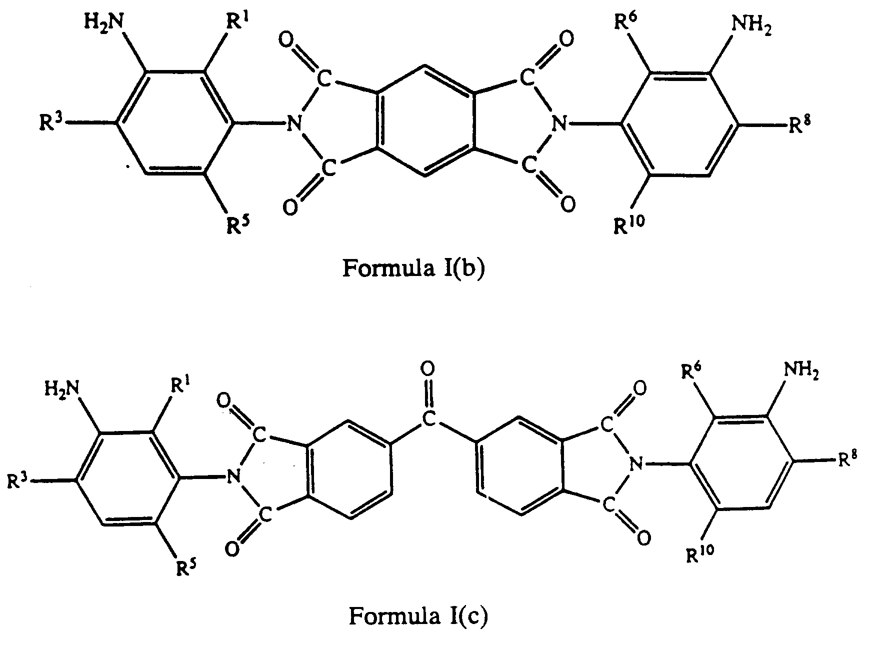 wherein any two of R¹, R³ or R⁵ are ethyl and the other is methyl and any two of R⁶, R⁸ and R¹⁰ are ethyl and the other is methyl.

When at least three of the groups R¹, R⁵, R⁶, and R¹⁰ in formula (Ia) are methyl or sterically larger groups the diaminobisimide compound is generally soluble in the solvents used commonly for prepregging, such as, for example, acetone and dichloromethane.

The relatively low melting point and good solubility of the compounds of formula (Ia) results in the preparation of the resins of the invention being simpler than conventional resins. For example, the epoxy resin of the invention comprising a compound of formula (Ia) as curing agent, together with polyepoxy resins such as DGEBA and TGDDM or mixtures thereof may be prepared by melt mixing using the following procedure:
1. Heating the epoxy resin formulation to a temperature sufficient to lower the viscosity to a level which enables the subsequent components to be incorporated satisfactorily in the mixer used.
2. Mixing in any toughening polymers and/or fillers required.
3. Incorporating the required quantity of diaminobisimide compound of formula (Ia).
4. Cooling the mixture as far as possible consistent with mixability before optionally adding a catalyst.

The epoxy resin formulations of the invention are generally liquids at processing temperatures in the range of 110°C to 170°C. This is not possible with impure diaminobisimides as discussed in D A Scola, *Polymer Composites*, **4**, 154 (1983). For example, when the compound of the formula (Ia) was derived from the reaction of Ethacure 100 (an amine wherein four of the R groups are ethyl , two are methyl and two are hydrogen) and pyromellitic acid i.e. CBH-103, the composition could be easily poured at 150°C.

Alternatively, a solvent-containing formulation of lower viscosity may be prepared by dissolving the epoxy resin in solvent, for example, acetone and mixing it with a solution of the diaminobisimide compound of the formula (I) curing agent in the same solvent. Other components such as fillers, catalysts, or rubber modifiers may be added to the mixture if desired. The amount of solvent preferably used is the minimum consistent with the flow properties required for the subsequent use.

However, in the preparation of an epoxy resin formulation suitable for forming the prepregs as described below the amount of solvent is preferably increased to reduce the viscosity of the mix and a solution of rubber modifiers is added to provide tackiness.

The epoxy resin formulation may be applied to a reinforcing material such as a uni-directional tape made from reinforcing fibre or to a woven fibre cloth either from a formulated solution such as described above (preferably with a lower aliphatic ketone or halogenated hydrocarbon solvent) by brushing or dipping, or from a hot melt. Application may be manual or by a machine process including those involving transfer from a precoated transfer medium. In the case of solution coating, after air drying the prepreg is preferably flash dried to remove the last of the solvent (usually <100°C for a short time) and stored at low temperature, preferably -10°C or less.

To produce a cured resin matrix, the diaminobisimide compounds of the formula (I) may be mixed with polyepoxides known per se in the art or with experimental polyepoxides and cured at temperatures of preferably up to 250°C. The curing reaction may be catalysed by the addition of BF₃⁻ ethylamine, BF₃⁻ benzylamine or other known catalysts to the composition.

Composite materials may be prepared from a mixture of the epoxy resin and reinforcing material by subjecting the epoxy resin formulation, after forming into the desired shape and size to a heating cycle to cure the resin.

Resin impregnated fibre materials or prepregs may be laid down by any existing method for preparing composite materials including vacuum bagging on a caul plate or on an appropriate tool. Curing can be carried out in an autoclave, hot platten press or other device. A suitable curing cycle is programmed linear temperature increase from 20°C to 180°C followed by 4 h at 180°C.

Alternatively, the epoxy resin formulations may be used for Resin Transfer Moulding.

The invention is further illustrated by the following non-limiting examples.

### Example 1 - Synthesis of 5,5'-carbonylbis[2-(4-aminophenyl)]-1H-isoindole-1,3(2H)-dione.

p- Phenylenediamine (2.16g) and 1,4-diazabicyclo[2.2.2]octane (1.0g) were dissolved in dimethylformamide (DMF) (50ml) in a round bottom flask under a nitrogen atmosphere and during stirring at 130°C, benzophenone tetracarboxylic dianhydride(BDTA) (3.22g) dissolved in DMF (50ml) was added slowly over 1 h to the diamine solution via a dropping funnel. After all the BDTA had been added the solution was heated just to boiling point. The solution became cloudy after about 1h and the solution was left to gently reflux under an air condenser for 6-8 h. The precipitate that formed was filtered off washed in ethanol and dried under vacuum over P₂O₅ at room temperature; the yield was 75%. The product was cleaned by extracting with ethanol in a soxhlet apparatus. The cleaned product was pale yellow solid which did not melt up to 400°C. Fourier Transform infra-red peaks at 3344, 3221, 1778, 1721, 1682, 713 cm⁻¹, and proton nmr peaks at 8.3, 8.1(imide), 6.7, 7.1(aminoaromatic) and 5.1(NH₂) ppm confirmed its structure.

### Example 2a - Synthesis of 2,6-bis(3-amino(methyldiethyl)phenyl)-benzo[1,2-c:4,5-c']-dipyrrole-1,3,5,7(1H,6H)-tetrone

Ethacure (Registered Trade Mark) 100 (Ethyl Corp) (3.31g) and 1,4-diazabicyclo[2.2.2]octane (1.0g) were dissolved in dimethylformamide(DMF) (50ml) in a round bottom flask under a nitrogen atmosphere and during stirring, pyromellitic dianhydride(PMDA) (2.02g) dissolved in DMF (50ml) was added slowly over 1 hour to the diamine solution via a dropping funnel. After all the PMDA had been added the solution was heated just to boiling point. The solution became very dark, almost black, after heating, the solution was left to gently reflux under an air condenser overnight (about 16 h). The solution was then concentrated and the concentrate added to a 50/50 methanol/water solution to form a precipitate. The precipitate that formed was filtered off and dried under vacuum over P₂O₅ at room temperature; the yield was 82%. The product can be cleaned by washing first with cold sodium bicarbonate solution then with cold ethanol. The cleaned product was orange in colour and had a DSC melting point of 304°C. Fourier Transform infra-red, proton and C¹³ nmr spectroscopy in CDCl₃ confirmed its structure. This diaminobisimide was soluble in acetone, chloroform and dichloromethane

### Example 2b

The compound above was also prepared in 90% yield by stirring molten Ethacure (Registered Trade Mark) 100 (2 mole) with dry pyromellitic dianhydride at room temperature and then slowly heating the mixture to 160°C under nitrogen for 2 h.

### Example 2c

The compound above was prepared in 86% yield by a further variation of the process of this invention in which the powdered salt of pyromellitic acid and Ethacure (Registered Trade Mark) 100 was suspended in water, the suspension degassed under vacuum then heated with stirring in a sealed vessel at 250°C and 3585 KPa for 4 hours.

### Example 3 - Synthesis of 5,5'-carbonylbis[2-(3-amino(methyl-dithiomethyl)phenyl)]-1H-isoindole-1,3(2H)-dione

Ethacure (Registered Trade Mark) 300 from Ethyl Corp (an isomeric mixture of methyldithiomethyl-metaphenylenediamines)(7.96g) and 1,4-diazabicyclo[2.2.2]octane (3.0g) were dissolved in dimethylformamide (DMF) (50ml) in a round bottom flask under a nitrogen atmosphere and during stirring, benzophenone tetracarboxylic dianhydride(BDTA) (2.02g) dissolved in DMF (50ml) was added slowly over 1 hour to the diamine solution via a dropping funnel. After all the BDTA had been added the solution was heated just to boiling point. After heating, the solution had become a clear orange, it was then left to gently reflux under an air condenser for about 4 h. The solution was then concentrated and the concentrate added to methanol to form a precipitate. The precipitate that formed was filtered off and dried under vacuum over P₂O₅ at room temperature; the yield was 73%. The product was sand colour and had a melting range of 175-200°C. Fourier Transform infra-red, proton and C¹³ nmr spectroscopy in CDCl₃ confirmed its structure.

### Example 4 - Synthesis of 2,6-bis(4-aminophenyl)-benzo[1,2-c:4,5-c']-dipyrrole-1,3,5,7 (2H,6H)tetrone

A mixture of pyromellitic acid (5.59g; 0.022mole) and 1,4-phenylenediamine (4.76g; 0.044mole) in water was degassed under vacuum and the air in the sealed vessel replaced with nitrogen. The mixture was heated with stirring at 250°C and 3585 KPa for 4h. When cool the fine powder was filtered off, washed with water, and dried to give the diaminobisimide 6.66g (84% yield calculated as monomer). The product had an infra-red spectrum similar to the product prepared in dimethyl formamide using pyromellitic dianhydride.

### Example 5 - Synthesis of 2,6-bis(3-amino-2,4,6-trimethylphenyl)-benzo[1,2-c:4,5-c']-dipyrrole-1,3,5,7(2H,6H)-tetrone

A mixture of pyromellitic acid (5.08g; 0.02 mole) and 2,4,6-trimethyl-1,3-phenylenediamine (6.00g; 0.04mole) in water was degassed under vacuum and the air in the sealed vessel replaced with nitrogen. The mixture was heated with stirring at 250C and 3585 KPa for 4h. When cool the fine powder was filtered off, washed with water, and dried to give the diaminobisimide 7.27g (70% yield calculated as monomer). The reaction product gave a 1H nmr spectrum in dimethylsulphoxide typical of a bisimide and confirmed the absence of starting materials, oligomers and side reaction products.

### Example 6 - A typical curing resin by melt blending

### (a) Preparation of the resin

A mixture of Araldite (Registered Trade Mark) MY720 (24.5g) and Epikote (Registered Trade Mark) 8283 IQ (a DGEBA type epoxy) (19.0g) was heated on a rotary evaporator at 100°C under 0.5mm vacuum for 1 hour. The diaminobisimide hardener CBH-104 (26.7g) was added and thoroughly mixed and then heated at 145-155°C/0.5mm for 30 minutes.

### (b) Curing

The neat resin mixture was transferred to heated moulds and cured at 135°C for 1.5h, 175°C for 2h and then 190°C for 3h and finally post cured at 205°C for 3h.

### Examples 7-22 - Other curable resin formulations

In other examples, the ratio of Araldite (Registered Trade Mark) MY720 to Epikote (Registered Trade Mark) 8283 IQ was varied. Some preparations used CBH-103 instead of CBH-104. Additives such as Ultem (Registered Trade Mark) 1000 and polyethersulfone (prepared according to A Noshay, M Matzner and C N Merriam, *J*. *Polym*. *Sci*: Part A-1, 9, 3147 (1971)) were dissolved in methylene chloride and added to the epoxy mixture, the methylene chloride removed under vacuum and then the mixture heated at 100°C/0.5mm for 1 hour. When methyl ethyl ketone was used it was added at the same time as the CBH-103 or CBH-104 and then removed under vacuum. Control examples were cured by heating at 100°C for 1h, 140°C for 1h and then 180°C for 4h. The diaminobisimide compounds CBH-103 and CBH-104 are somewhat less reactive than DDS as shown by the higher peak temperatures for DSC cures listed in Table 1 and therefore benefit from a post cure at temperatures over 200°C. Alternatively, a catalyst may be used.

Table 2 shows that when the polyepoxide is a mixture of MY720 and DGEBA, the formulations of the invention when cured have much higher Tg values than the corresponding prior art composition using DDS as hardener. In addition, the new cured resins have the same or greater toughness which can be further increased with the addition of thermoplastics such as Ultem (Registered Trade Mark) 1000 or polyethersulfones.

### Examples 23 and 24 - Preparation of typical laminates

### (a) Preparation of prepregs

Resins wherein the epoxy component was a 85% MY720 / 15% DGEBA mixture, containing differing levels of CBH-103 diaminobisimide were dissolved in methyl ethyl ketone and painted on to Fiberite High Performance Structural W322 woven carbon fibre cloth. The prepreg was dried in a stream of warm air for 10 minutes and then "B" staged in an oven at 90°C for 60 sec. The prepregs had a mean resin content of 38%.

### (b) Laminate production

A 20 ply laminate was laid up according to BSS 7273 from each prepreg and cured in an autoclave at 100°C for 1h, 140°C for 1h and then 180°C for 4h under a pressure of 620 kPa. Mode I interlaminar fracture toughness of each 20 ply cloth laminate was tested according to BSS 7273; the values obtained are given in Table 3, which shows that laminates (composites) prepared from the cured resins of the invention have mode I interlaminar fracture toughness values which exceed the minimum value of 175 Jm⁻² (area) specified in BMS 8-256F.

### Example 25 - Hot/wet resistance of the cured resins and laminates

Water in vapour or liquid form has a deleterious effect on some cured resins, lowering Tg and hence use temperature ceiling or causing delamination and microcracking in aircraft laminates due to expansion and contraction of the wet laminate with changing environmental conditions. The possible effect of water on a cured composite is assessed by the hot water uptake test which involves measuring water uptake after soaking samples in deionized water at 71°C for up to 37 days. Table 4 shows that the water uptake of the formulations of the invention when cured is less than those cured with DDS. The water uptake of the corresponding composite is less than that of a commercial toughened composite and similar to that of the experimental DDS cured laminate.

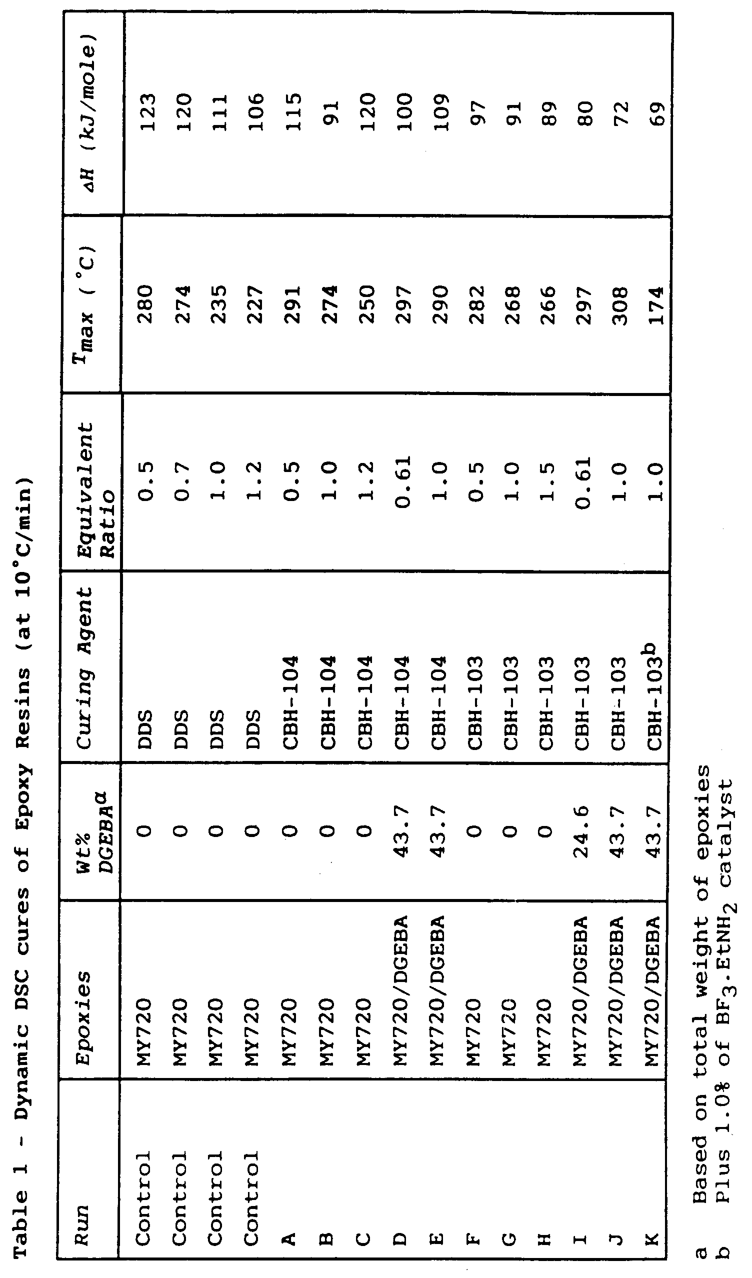

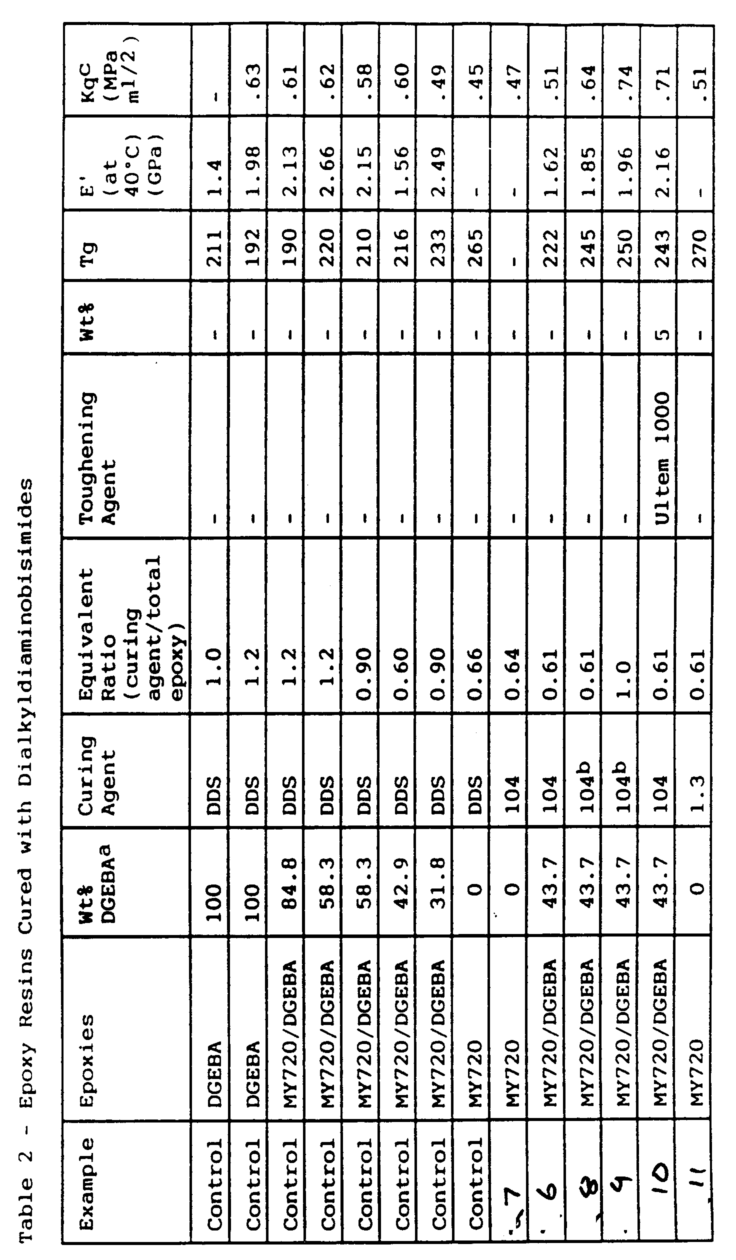

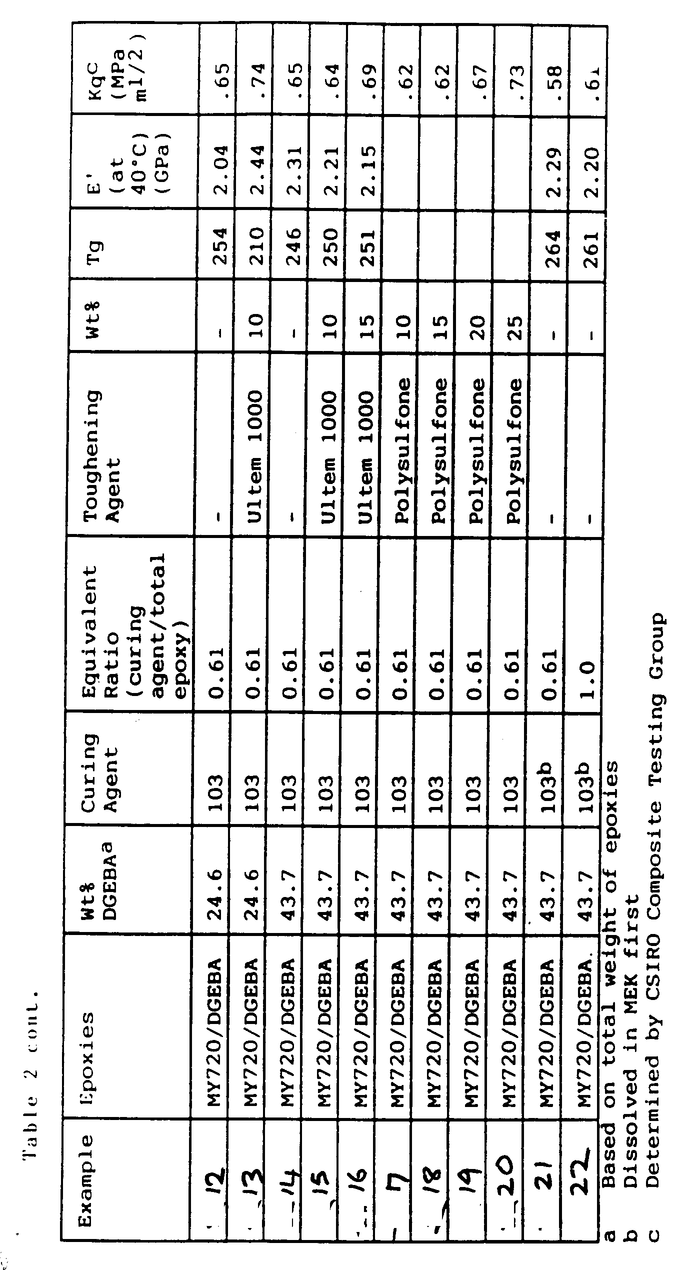

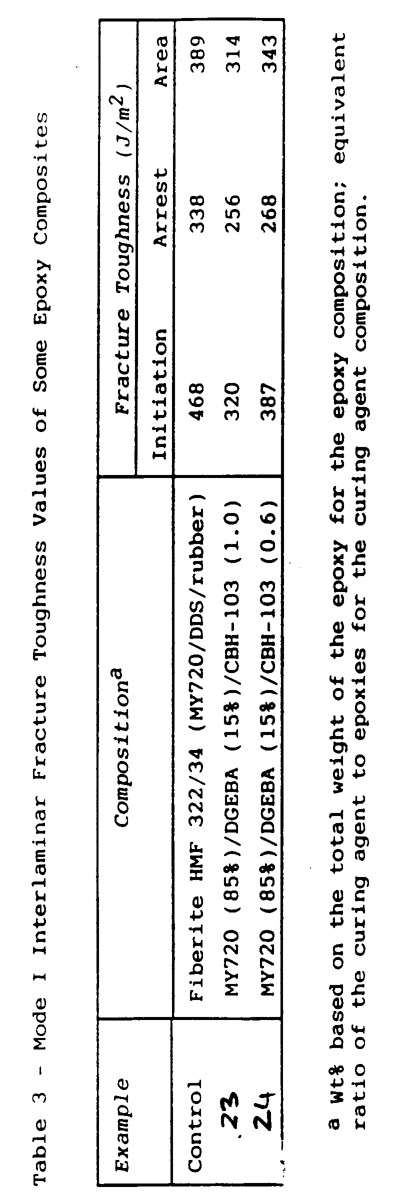

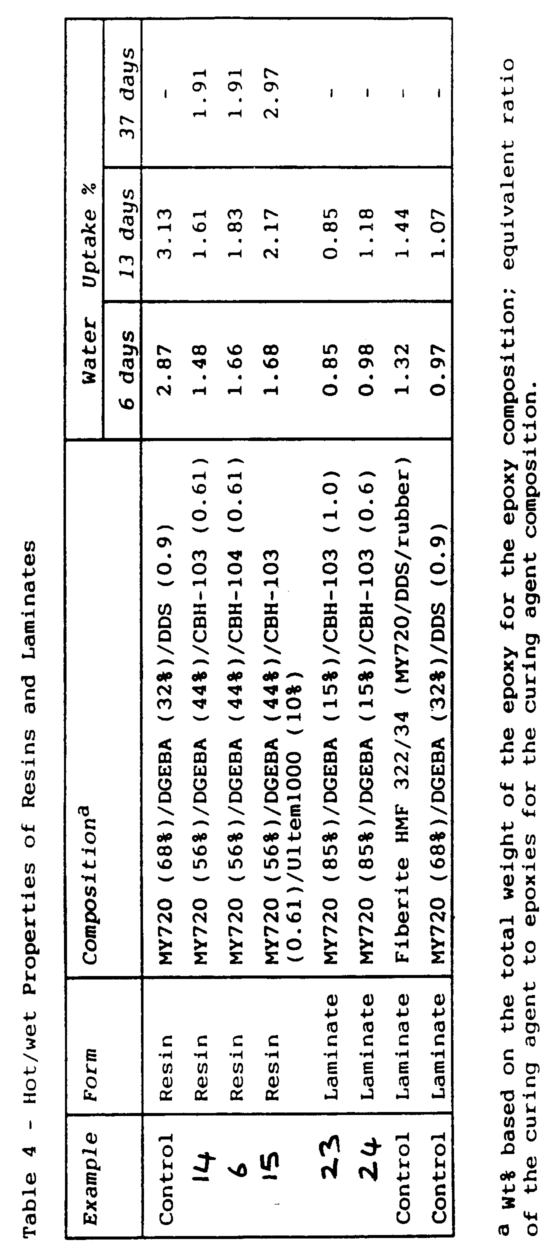

## Claims

1. A method for the preparation of a diaminobisimide compound of the formula (I) substantially free of oligomers: 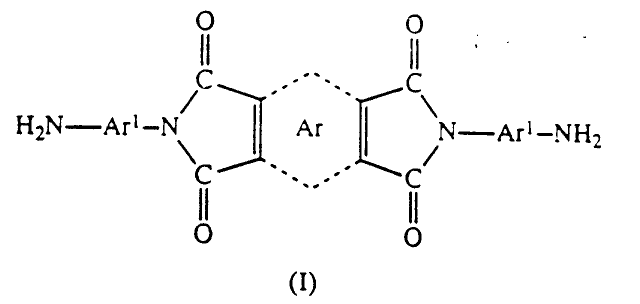 wherein
Ar¹ is an optionally substituted aromatic residue which provides for good conjugation between the nitrogen containing groups; and
Ar is an optionally substituted aromatic residue
characterized in that at least two molar proportions of an aromatic diamine of the formula (II)
H₂N-Ar¹-NH₂ (II)
wherein Ar¹ is as defined above,
are heated with one molar proportion of an aromatic tetracarboxylic acid of the formula (III) or the corresponding dianhydride,
(HOOC)₂Ar(COOH)₂ (III)
wherein Ar is as defined above,
optionally in the presence of a polar solvent and optionally including 0.1 to 2 molar proportions of a tertiary amine,
with the proviso that when a dianhydride is used either:
(i) 0.1 to 2 molar proportions of a tertiary amine are included, or
(ii) the aromatic diamine is reacted with a higher melting aromatic dianhydride in the molten state such that the reacting diamine melt is always in high molar excess.

2. A method as claimed in Claim 1, characterised in that at least two molar proportions of aromatic diamine of the formula (II) are reacted with one mole of an aromatic tetracarboxylic acid of the formula (III) in the presence of water as the polar solvent to produce the diaminodiacid salt of the formula 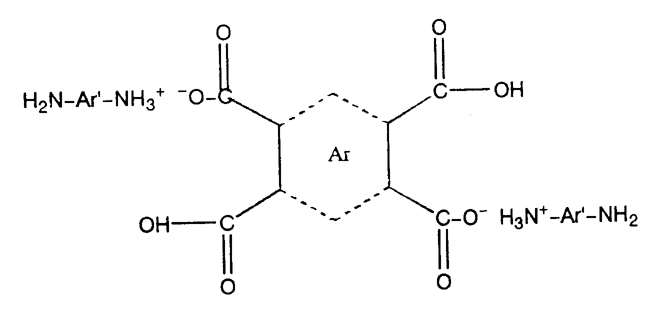 which is then heated to between 80°C and 250°C.

3. A method as claimed in Claim 1, characterised in that the aromatic diamine of the formula (II) is sterically hindered and is reacted with a higher melting aromatic dianhydride in the molten state in such manner that the reacting diamine melt is always in a high molar excess.

4. A method as claimed in Claim 1, characterised in that the aromatic diamine of the formula (II) is sterically hindered and reacted with a higher melting aromatic tetracarboxylic acid of the formula (III) in the molten state in such manner that the reacting diamine melt is always in a high molar excess.

5. Use of a diaminobisimide compound of the formula (I), substantially free from oligomers, as defined in Claim 1, as a curing agent in epoxy resin formulation.

6. A diaminobisimide compound of the formula (Ia) substantially free from oligomers 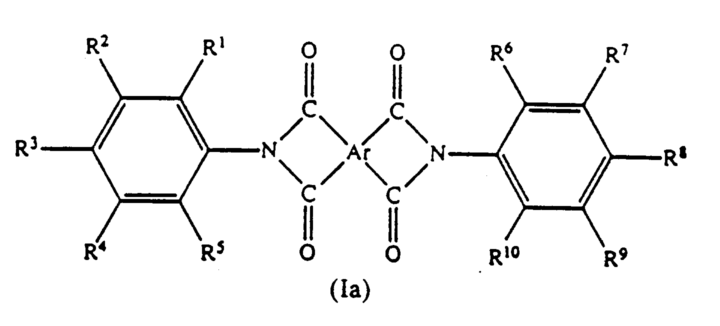 wherein
R¹ to R¹⁰ are the same or different and each may be selected from hydrogen, alkyl, alkylthio, alkoxy, dialkylamino, alkylamino and amino; and
Ar is an optionally substituted aromatic residue,
with the proviso that at least one of R¹ to R⁵ and R⁶ to R¹⁰ is an alkyl group and at least one of R² to R⁴ and R⁷ to R⁹ is an amino group.

7. A diaminobisimide compound as claimed in Claim 6, characterised in that at least three of R¹, R⁵, R⁶ and R¹⁰ are methyl or sterically larger groups.

8. Use of a diaminobisimide compound of the formula (Ia), substantially free from oligomers, as claimed in Claim 6 or 7, as a curing agent in epoxy resin formulation.

9. An epoxy resin formulation comprising a mixture of a curing agent and one or more polyepoxides, characterized in that the curing agent is a compound of the formula (I) substantially free from oligomer, as claimed in Claim 1.

10. An epoxy resin formulation comprising a mixture of a curing agent and one or more polyepoxides, characterized in that the curing agent is a compound of the formula (Ia) substantially free from oligomer as claimed in Claim 6 or Claim 7.

11. A cured epoxy resin, characterized in that the cured epoxy resin is produced by heating the formulation as claimed in Claim 9 or Claim 10 at a temperature up to 250°C.

12. An impregnated fibre reinforced material, characterized in that the fibre reinforcements are coated with an epoxy resin formulation as claimed in Claim 9 or Claim 10.

13. An advanced composite material comprising an assembly of reinforcing fibres in a matrix of cured epoxy resin, characterized in that the cured epoxy resin is as claimed in Claim 11.

## Patentansprüche

1. Verfahren zur Herstellung einer Diaminobisimidverbindung der Formel (I), die im wesentlichen frei von Oligomeren ist: 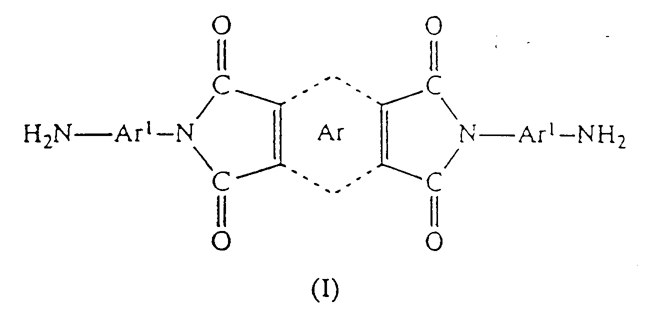 worin
Ar¹ ein gegebenenfalls substituierter aromatischer Rest ist, der eine gute Konjugation zwischen den Stickstoff enthaltenden Gruppen verursacht und
Ar ein gegebenenfalls substituierter aromatischer Rest ist,
dadurch gekennzeichnet, daß mindestens zwei molare Anteile eines aromatischen Diamins der Formel (II)
H₂N-Ar¹-NH₂ (II)
worin Ar¹ wie vorstehend definiert ist,
mit einem molaren Anteil einer aromatischen Tetracarbonsäure der Formel (III) oder des entsprechenden Dianhydrids
(HOOC)₂Ar(COOH)₂ (III)
worin Ar wie vorstehend definiert ist,
gegebenenfalls in Gegenwart eines polaren Lösungsmittels, das gegebenenfalls 0,1 bis 2 molare Anteile eines tertiären Amins einschließt, erhitzt werden,
mit der Maßgabe, daß dann, wenn ein Dianhydrid verwendet wird, entweder
(i) 0,1 bis 2 molare Anteile eines tertiären Amins eingeschlossen sind oder
(ii) das aromatische Diamin mit einem höherschmelzenden aromatischen Dianhydrid im geschmolzenen Zustand derart umgesetzt wird, daß die reagierende Diaminschmelze immer in einem hohen molaren Überschuß vorliegt.

2. Verfahren nach Anspruch 1, dadurch gekennzeichnet, daß mindestens zwei molare Anteile des aromatische Diamins der Formel (II) mit einem Mol einer aromatischen Tetracarbonsäure der Formel (III) in Gegenwart von Wasser als polares Lösungsmittel umgesetzt werden, wobei das Diaminodisäuresalz der Formel 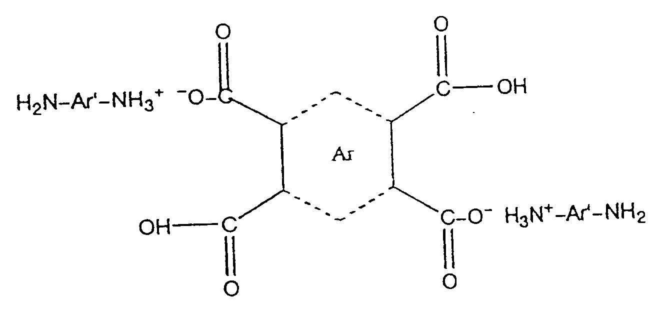 gebildet wird, welches dann auf 80°C bis 250°C erhitzt wird.

3. Verfahren nach Anspruch 1, dadurch gekennzeichnet, daß das aromatische Diamin der Formel (II) sterisch gehindert ist und in der Weise mit einem höher schmelzenden aromatischen Dianhydrid in geschmolzenem Zustand umgesetzt wird, daß die reagierende Diaminschmelze immer in einem hohen molaren Überschuß vorliegt.

4. Verfahren nach Anspruch 1, dadurch gekennzeichnet, daß das aromatische Diamin der Formel (II) sterisch gehindert ist und in der Weise mit einer höher schmelzenden aromatischen Tetracarbonsäure der Formel (III) im geschmolzenen Zustand umgesetzt wird, daß die reagierende Diaminschmelze immer in einem hohen molaren Überschuß vorliegt.

5. Verwendung einer Diaminobisimidverbindung der Formel (I), die im wesentlichen frei von Oligomeren ist, gemäß der Definition in Anspruch 1, als Härtungsmittel in einer Epoxyharzformulierung.

6. Diaminobisimidverbindung der Formel (Ia), die im wesentlichen frei von Oligomeren ist worin 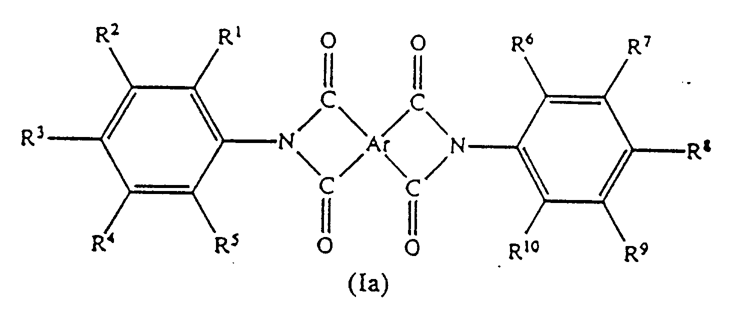
R¹ bis R¹⁰ gleich oder verschieden sind und jeweils unter Wasserstoff, Alkyl, Alkylthio, Alkoxy, Dialkylamino, Alkylamino und Amino ausgewählt sind und
Ar ein gegebenenfalls substituierter aromatischer Rest ist,
mit der Maßgabe, daß mindestens eines von R¹ bis R⁵ und R⁶ bis R¹⁰ eine Alkylgruppe ist und mindestens eines von R² bis R⁴ und R⁷ bis R⁹ eine Aminogruppe ist.

7. Diaminobisimidverbindung nach Anspruch 6, dadurch gekennzeichnet, daß mindestens drei von R¹, R⁵, R⁶ und R¹⁰ Methylgruppen oder sterisch größere Gruppen darstellen.

8. Verwendung einer Diaminobisimidverbindung der Formel (Ia), die im wesentlichen frei von Oligomeren ist, nach Anspruch 6 oder 7, als Härtungsmittel in einer Epoxyharzformulierung.

9. Epoxyharzformulierung, enthaltend ein Gemisch aus einem Härtungsmittel und einem oder mehr Polyepoxiden, dadurch gekennzeichnet, daß das Härtungsmittel eine im wesentlichen von Oligomeren freie Verbindung der Formel (I) gemäß Anspruch 1 ist.

10. Epoxyharzformulierung, die ein Gemisch aus einem Härtungsmittel und einem oder mehr Polyepoxiden enthält, dadurch gekennzeichnet, daß das Härtungsmittel eine im wesentlichen von Oligomeren freie Verbindung der Formel (Ia) gemäß Anspruch 6 oder 7 ist.

11. Gehärtetes Epoxyharz, dadurch gekennzeichnet, daß das gehärtete Epoxyharz durch Erhitzen einer Formulierung gemäß Anspruch 9 oder Anspruch 10 auf eine Temperatur bis zu 250°C gebildet worden ist.

12. Mit imprägnierten Fasern verstärktes Material, dadurch gekennzeichnet, daß die Faserverstärkungen mit einer Epoxyharzformulierung gemäß Anspruch 9 oder Anspruch 10 überzogen sind.

13. Hochwertiges Verbundmaterial, das eine Anordnung von Verstärkungsfasern in einer Matrix aus gehärtetem Epoxyharz umfaßt, dadurch gekennzeichnet, daß das gehärtete Epoxyharz wie gemäß Anspruch 11 ist.

## Revendications

1. Procédé de préparation d'un composé diaminobisimide de formule (I) pratiquement complètement exempt d'oligomères : 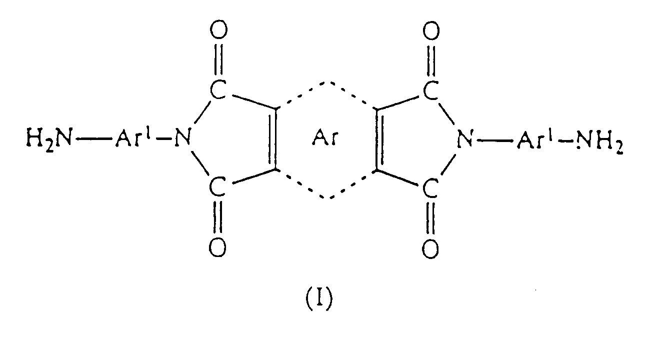 dans laquelle
Ar¹ est un groupe aromatique éventuellement substitué qui apporte une bonne conjugaison entre les groupes renfermant de l'azote; et
Ar est un groupe aromatique éventuellement substitué
caractérisé en ce qu'on chauffe au moins deux proportions molaires d'une diamine aromatique de formule (II)
H₂N-Ar¹-NH₂ (II)
dans laquelle Ar¹ est tel que défini ci-dessus,
avec une proportion molaire d'un acide tétracarboxylique aromatique de formule (III) ou du dianhydride correspondant :
(HOOC)₂Ar(COOH)₂ (III)
dans laquelle Ar est tel que défini ci-dessus,
éventuellement en présence d'un solvant polaire et comprenant éventuellement 0,1 à 2 proportions molaires d'une amine tertiaire,
à condition que, lorsqu'on utilise un dianhydride, soit :
(i) on inclue 0,1 à 2 proportions molaires d'une amine tertiaire, ou
(ii) la diamine aromatique soit mise à réagir avec un dianhydride aromatique de plus haut point de fusion, à l'état fondu, de sorte que la diamine fondue en cours de réaction soit toujours en fort excès molaire.

2. Procédé tel que revendiqué à la revendication 1, caractérisé en ce qu'au moins deux proportions molaires de diamine aromatique de formule (II) sont mises à réagir avec une mole d'un acide aromatique tétracarboxylique de formule (III) en présence d'eau comme solvant polaire pour produire le sel de diaminodiacide de formule 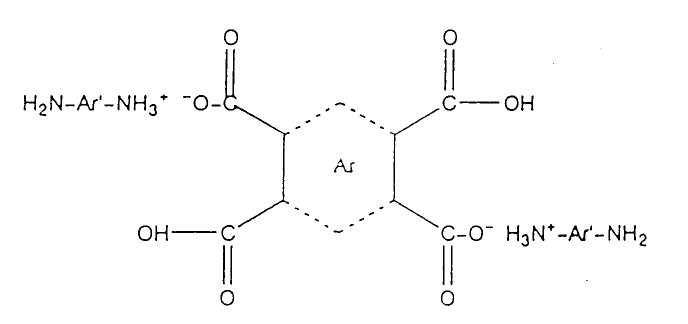 qui est ensuite chauffé entre 80°C et 250°C.

3. Procédé tel que revendiqué à la revendication 1, caractérisé en ce que la diamine aromatique de formule (II) est à empêchement stérique et est mise à réagir à l'état fondu avec un dianhydride aromatique de point de fusion supérieur, de telle manière que la diamine fondue en cours de réaction soit toujours en fort excès molaire.

4. Procédé tel que revendiqué à la revendication 1, caractérisé en ce que la diamine aromatique de formule (II) est à empêchement stérique et est mise à réagir avec un acide tétracarboxylique aromatique de formule (III) à l'état fondu de telle manière que la diamine fondue en cours de réaction soit toujours en fort excès molaire.

5. Utilisation d'un composé diaminobisimide de formule (I), pratiquement complètement exempt d'oligomères, tel que défini à la revendication 1, comme agent durcisseur dans une formulation de résine époxy.

6. Composé diaminobisimide de formule (Ia) pratiquement complètement exempt d'oligomères 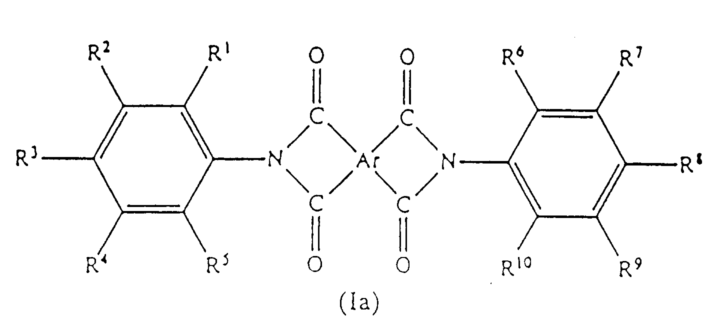 dans laquelle
R¹ à R¹⁰ sont identiques ou différents et chacun d'eux peut être choisi parmi un atome d'hydrogène, un groupe alkyle, alkylthio, alcoxy, dialkylamino, alkylamino et amino; et
Ar est un groupe aromatique éventuellement substitué,
à condition qu'au moins l'un des groupes R¹ à R⁵ et R⁶ à R¹⁰ soit un groupe alkyle et qu'au moins l'un des groupes R² à R⁴ et R⁷ à R⁹ soit un groupe amino.

7. Composé diaminobisimide tel que revendiqué à la revendication 6, caractérisé en ce qu'au moins trois des groupes R¹, R⁵, R⁶ et R¹⁰ sont des groupes méthyle ou stériquement plus gros.

8. Utilisation d'un composé diaminobisimide de formule (Ia), pratiquement complètement exempt d'oligomères, tel que revendiqué à la revendication 6 ou 7, comme agent durcisseur dans une formulation de résine époxy.

9. Formulation de résine époxy comprenant un mélange d'un agent durcisseur et d'un ou plusieurs polyépoxydes, caractérisée en ce que l'agent durcisseur est un composé de formule (I) pratiquement complètement exempt d'oligomères, tel que revendiqué à la revendication 1.

10. Formulation de résine époxy comprenant un mélange d'un agent durcisseur et d'un ou plusieurs polyépoxydes, caractérisée en ce que l'agent durcisseur est un composé de formule (Ia) pratiquement complètement exempt d'oligomères, tel que revendiqué à la revendication 6 ou à la revendication 7.

11. Résine époxy durcie, caractérisée en ce que la résine époxy durcie est produite par chauffage de la formulation telle que revendiquée à la revendication 9 ou à la revendication 10 à une température allant jusqu'à 250°C.

12. Matériau imprégné renforcé par une fibre, caractérisé en ce que les renforcements de fibre sont recouverts avec une formulation de résine époxy telle que revendiquée à la revendication 9 ou à la revendication 10.

13. Matériau composite avancé comprenant un assemblage de fibres de renforcement dans une matrice de résine époxy durcie, caractérisé en ce que la résine époxy est telle que revendiquée à la revendication 11.
